# EUROPEAN PATENT SPECIFICATION

(11) **EP 2 986 325 B1**
(45) Date of publication and mention of the grant of the patent: **26.06.2019**
(21) Application number: 14854074.3
(22) Date of filing: 14.10.2014
(51) Int. Cl.: A61K 31/235, A61K 31/192, G01N 33/68, G01N 33/50

(54) **METHODS OF TREATING UREA CYCLE DISORDERS**
VERFAHREN ZUR BEHANDLUNG VON HARNSTOFFZYKLUSSTÖRUNGEN
PROCÉDÉS DE TRAITEMENT DE TROUBLES DU CYCLE DE L'URÉE

(30) Priority: 14.10.2013 US 201361890827 P; 29.08.2014 US 201462044168 P
(43) Date of publication of application: 24.02.2016
(73) Proprietor: Immedica Pharma AB, 113 29 Stockholm (SE)
(72) Inventor: MOKHTARANI, Masoud, Walnut Creek, CA 94598 (US); SCHARSCHMIDT, Bruce, San Francisco, CA 94127 (US)
(74) Representative: Zacco Sweden AB
(86) International application number: PCT/US2014/060543
(87) International publication number: WO 2015/057747

(56) References cited:
- WO-A1-2011/011781
- WO-A2-2013/048558
- WO-A2-2013/048558
- US-A1- 2012 022 157
- GREGORY M ENNS ET AL: "Survival after Treatment with Phenylacetate and Benzoate for Urea-Cycle Disorders A BS T R AC T", THE NEW ENGLAND JOURNAL OF MEDICINE, vol. 356, no. 22, 31 May 2007 (2007-05-31) , pages 2282-2292, XP055148817,
- JON P. R. MONTELEONE ET AL: "Population Pharmacokinetic Modeling and Dosing Simulations of Nitrogen-Scavenging Compounds: Disposition of Glycerol Phenylbutyrate and Sodium Phenylbutyrate in Adult and Pediatric Patients with Urea Cycle Disorders", JOURNAL OF CLINICAL PHARMACOLOGY., vol. 53, no. 7, 15 June 2013 (2013-06-15), pages 699-710, XP055244763, US ISSN: 0091-2700, DOI: 10.1002/jcph.92
- MONTELEONE ET AL.: 'Population Pharmacokinetic Modeling and Dosing Simulations of Nitrogen-Scavenging Compounds: Disposition of Glycerol Phenylbutyrate and Sodium Phenylbutyrate in Adult and Pediatric Patients with Urea Cycle Disorders.' J CLIN PHARMACOL. vol. 53, no. 7, July 2013, pages 699 - 710, XP055244851 Retrieved from the Internet: <URL:http://www.ncbi.nlm.nih.gov/pmc/articl es/PMC3923458/pdf/nihms536149.pdf> [retrieved on 2014-12-15]

## Description

### BACKGROUND

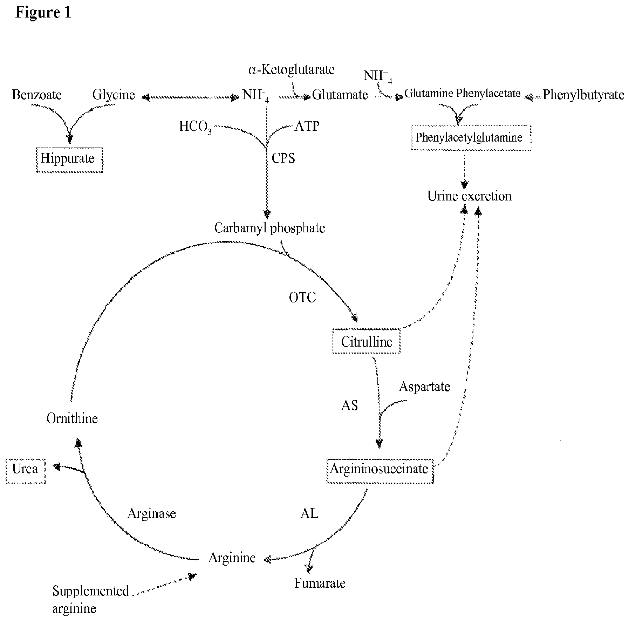

Urea cycle disorders (UCDs) are nitrogen retention disorders associated with elevated ammonia levels. UCDs include several inherited deficiencies of enzymes or transporters necessary for the synthesis of urea from ammonia, including enzymes involved in the urea cycle. The urea cycle is depicted in Figure 1, which also illustrates how certain ammonia-scavenging drugs act to assist in elimination of excessive ammonia. With reference to Figure 1, N-acetyl glutamine synthetase (NAGS)-derived *N*-acetylglutamate binds to carbamyl phosphate synthetase (CPS), which activates CPS and results in the conversion of ammonia and bicarbonate to carbamyl phosphate. In turn, carbamyl phosphate reacts with ornithine to produce citrulline in a reaction mediated by ornithine transcarbamylase (OTC). A second molecule of waste nitrogen is incorporated into the urea cycle in the next reaction, mediated by arginosuccinate synthetase (ASS), in which citrulline is condensed with aspartic acid to form argininosuccinic acid. Argininosuccinic acid is cleaved by argininosuccinic lyase (ASL) to produce arginine and fumarate. In the final reaction of the urea cycle, arginase (ARG) cleaves arginine to produce ornithine and urea. Of the two atoms of nitrogen incorporated into urea, one originates from free ammonia (NH₄⁺) and the other from aspartate. UCD individuals born with no meaningful residual urea synthetic capacity typically present in the first few days of life (neonatal presentation). Individuals with residual function typically present later in childhood or even in adulthood, and symptoms may be precipitated by increased dietary protein or physiological stress *(*e.g., intercurrent illness).

Subjects with UCDs that are not adequately controlled by dietary restriction of protein and/or dietary supplements are often treated with nitrogen scavenging agents, which provide an alternate pathway to urea for excretion of waste nitrogen (Brusilow 1980; Brusilow 1991). These nitrogen scavenging agents include sodium phenylbutyrate (NaPBA, approved in the United States as BUPHENYL® and in Europe as AMMONAPS®) and glyceryl tri-[4-phenylbutyrate] (HPN-100, GT4P, glycerol PBA, approved in the United States as RAVICTI®; see U.S. Patent No. 5,968,979). NaPBA is a phenylacetic acid (PAA) prodrug, while HPN-100 is a prodrug of PBA and a pre-prodrug of PAA.

HPN-100 and NaPBA share the same general mechanism of action: PBA is converted to PAA via beta oxidation, and PAA is conjugated enzymatically with glutamine to form phenylacetylglutamine (PAGN), which is excreted in the urine. The structures of PBA, PAA, and PAGN are set forth below.

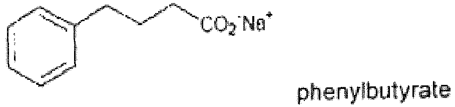

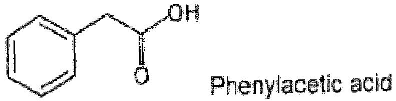

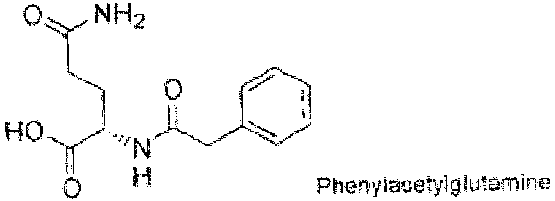

The clinical benefit of NaPBA and HPN-100 with regard to nitrogen retention disorders derives from the ability of PAGN to effectively replace urea as a vehicle for waste nitrogen excretion and/or to reduce the need for urea synthesis (Brusilow 1991; Brusilow 1993). Because each glutamine contains two molecules of nitrogen, the body rids itself of two waste nitrogen atoms for every molecule of PAGN excreted in the urine. Therefore, two equivalents of nitrogen are removed for each mole of PAA converted to PAGN. PAGN represents the predominant terminal metabolite, and one that is stoichiometrically related to waste nitrogen removal, a measure of efficacy in the case of nitrogen retention states. The difference between HPN-100 and NaPBA with respect to metabolism is that HPN-100 is a triglyceride and requires digestion, presumably by pancreatic lipases, to release PBA (McGuire 2010). Enns et al. (New England Journal of Medicine, vol. 356, no. 22, 31 May 2007, pages 2282-2292) discloses the "results of a 25-year, open label, uncontrolled study of sodium phenylacetate and sodium benzoate therapy (Ammonul, Ucyclyd Pharma) in 299 patients with urea cycle disorders in whom there were 1181 episodes of acute hyperammonemia". Monteleone et al. (Journal of Clinical Pharmacology, vol. 53, no. 7, 15 Juen 2013, pages 699-710) discloses that "sodium phenylbutyrate and glycerol phenylbutyrate mediate waste nitrogen excretion in the form of urinary PAGN (uPAGN) in patients with urea cycle disorders (UCDs)". WO 2013/048558 A2 provides "methods for evaluating daily ammonia exposure based on a single fasting ammonia blood level measurement, as well as methods that utilize this technique to adjust the dosage of a nitrogen scavenging drug, determine whether to administer a nitrogen scavenging drug, and treat nitrogen retention disorders". WO 2011/011781 A1 discloses a "method of modulating plasma levels of branched chain amino acids and branched chain alpha-keto acids... wherein an ammonia scavenger compound or a salt thereof... is administered to an individual in need thereof".

### SUMMARY OF THE INVENTION

The invention relates to a method of determining an effective dosage of glyceryl tri-[4-phenylbutyrate] for treating a urea cycle disorder in a subject aged 6 to 17 years old in need thereof comprising: calculating a body surface area (BSA) for the subject; and comparing the BSA to a predetermined threshold value which is 1.3 m², wherein the effective dosage is a first dosage of 7.3 to 8.79 g/m²/day if the BSA is at or above the predetermined threshold value or a second dosage of 9 to 9.9 g/m²/day if the BSA is below the predetermined threshold value. The invention also relates to glyceryl tri-[4-phenylbutyrate] for use in a method for treating a urea cycle disorder (UCD) in a subject aged 6 to 17 years old in need thereof, wherein the method includes the steps of: calculating a body surface area (BSA) for the subject, comparing the BSA to a predetermined threshold value which is 1.3 m², and administering a first dosage of 7.3 to 8.79 g/m²/day of glyceryl tri-[4-phenylbutyrate] if the BSA is at or above the predetermined threshold value or a second dosage of 9 to 9.9 g/m²/day of glyceryl tri-[4-phenylbutyrate] if the BSA is below the predetermined threshold value.

Preferred aspects of the invention are defined in the dependent claims.

### BRIEF DESCRIPTION OF DRAWINGS

Figure 1: The urea cycle and how certain nitrogen-scavenging drugs may assist in elimination of excessive ammonia.

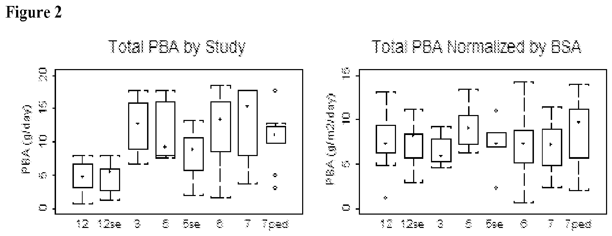

Figure 2: Summary of dosing information across the HPN-100 UCD studies HPN-100-012 switchover (12), HPN-100-012 safety extension (12se), UP1204-003 (3), HPN-100-005 switchover (5), HPN-100-005 safety extension (5se), HPN-100-006 (6), and HPN-100-007 (7, 7ped are ages 6 to 17). The bottom and top of a box represent the 25th and 75th percentiles, respectively, of distribution. The ends of the whiskers represent roughly the 5th and 95th percentiles. The black dot is the median dose.

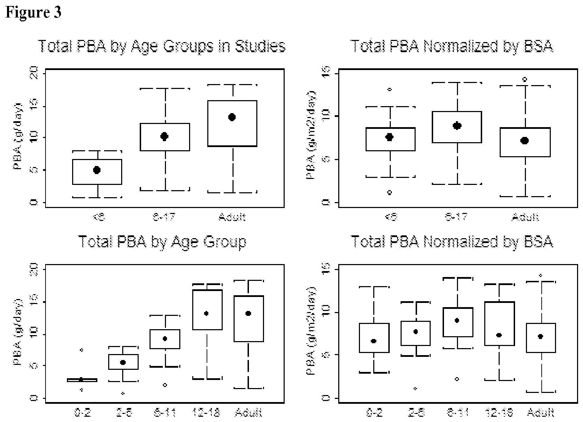

Figure 3: Summary of dosing information across adult (n=65) and pediatric (n=49) populations. Left panels show dosage by age group, right boxes show dosage normalized by BSA. The bottom and top of a box represent the 25th and 75th percentiles, respectively, of distribution. The ends of the whiskers represent roughly the 5th and 95th percentiles. The black dot is the median dose.

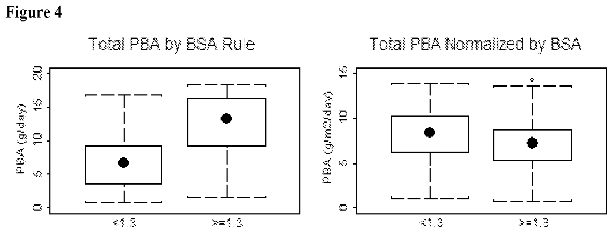

Figure 4: Summary of dosing information across adult (n=65) and pediatric (n=49) populations stratified by BSA < 1.3 m² or BSA ≥ 1.3 m² among pediatric and adult patients, which represents the lowest BSA value for the adult population approximate demarcation for age groups 6-11 and 12-17. The bottom and top of a box represent the 25th and 75th percentiles, respectively, of distribution. The ends of the whiskers represent roughly the 5th and 95th percentiles. The black dot is the median dose.

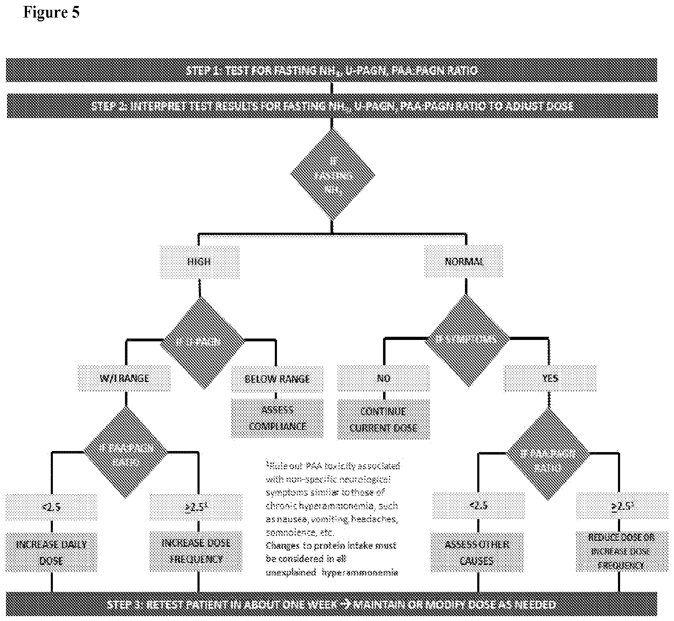

Figure 5: Sample dosing regimen for treatment with PAA prodrug.

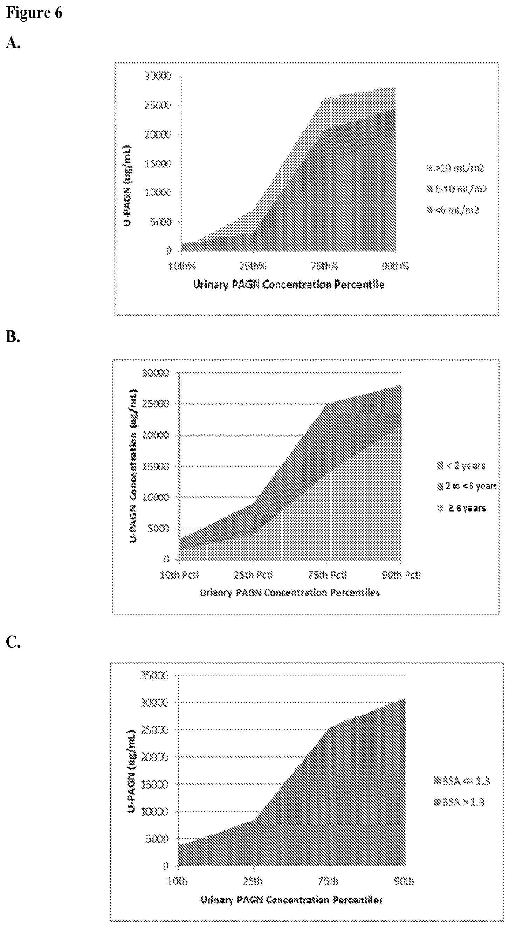

Figure 6: Urinary PAGN (U-PAGN) concentrations based on (A) dose of PAA prodrug, (B) age, or (C) BSA. (A) Percentiles are based on dose of PAA prodrug. Medium grey represents PAA prodrug dose < 6 mL/m², dark grey represents PAA prodrug dose 6-10 mL/m², and light grey represents PAA prodrug dose > 10 mL/m². (B) Percentiles are based on subject's age. Medium grey represents subjects younger than 2 years of age, dark grey represents subjects 2 to 6 years of age, and light grey represents subjects 6 years of age and older. (C) Percentiles of U-PAGN concentrations based on BSA. Light grey represents BSA greater than 1.3 m² and dark grey represents BSA less than or equal to 1.3 m².

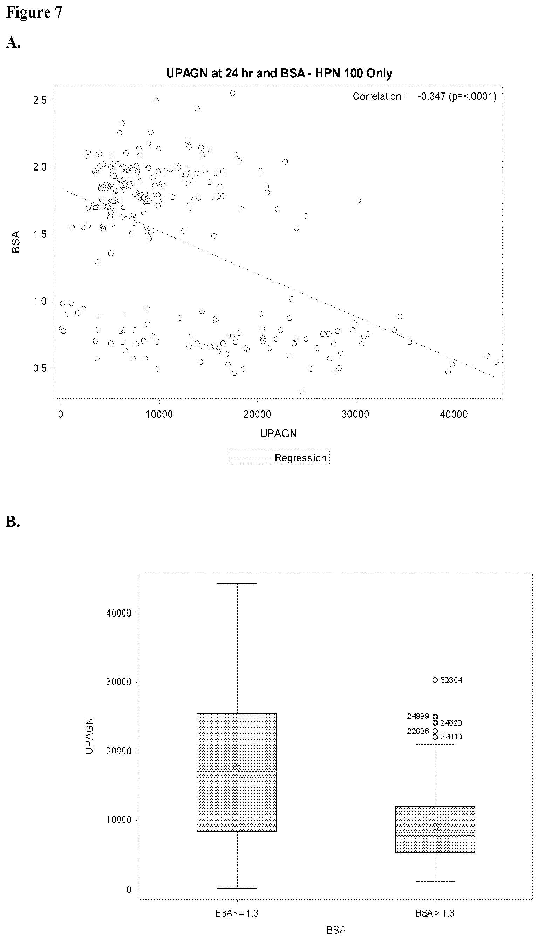

Figure 7: Correlation of U-PAGN concentrations and BSA. (A) A regression analysis showing a negative correlation of urinary PAGN concentrations with BSA. (B) Box plots showing BSA ≤ 1.3 m² (left box plot) or BSA > 1.3 m² (right box plot) among patients. The bottom and top of a box represent the 25th and 75th percentiles, respectively, of distribution. The ends of the whiskers represent roughly the 5th and 95th percentiles. The open-diamond black dot is the median concentration.

### DETAILED DESCRIPTION

The following description of the invention is merely intended to illustrate various embodiments of the invention, which is defined in the claims.

PAA prodrugs currently approved for the treatment of UCDs include NaPBA (BUPHENYL®) and HPN-100 (RAVICTI®). NaPBA is approved for use in chronic management of both neonatal- and late-onset UCDs involving deficiencies of carbamylphosphate synthetase, ornithine transcarbamylase, or argininosuccinic acid synthetase in adult and pediatric subjects. The current recommended dosage of NaPBA is 450-600 mg/kg/day for subjects weighing less than 20 kg or 9.9-13.0 g/m²/day in larger subjects. HPN-100 has recently been approved for use in chronic management of UCDs in adult and pediatric subjects two years of age or older. The current recommended dosage of HPN-100 for subjects who have not previously received a PBA-based drug is 5 to 12.4 g/m²/day (4.5 to 11.2 mL/m²/day), or 4.5 mL/m²/day for subjects with some residual enzyme activity who are not adequately controlled with dietary restriction. For subjects transitioning from treatment with NaPBA, the current recommended dosage is the daily dosage of NaPBA (in g) multiplied by 0.86.

The recommended dosages for HPN-100 are based on data from multiple clinical studies. In the phase 1 study UP 1204-0001, 24 healthy male adult subjects were administered a single dose of HPN-100 or NaPBA at a dosage equivalent to 3 g/m² of PBA (McGuire 2010). In another phase 1 study, UP 1204-0002, 32 adults (8 healthy and 24 with Child-Pugh grade A, B, or C cirrhosis) were administered HPN-100 BID at a dosage of 100 mg/kg (McGuire 2010; Ghabril Digestive Disease Week). These phase 1 studies established the safety of HPN-100 administration at dosages equivalent to the highest approved NaPBA dosage of 20 g/day (Lee 2010).

In the phase 2 study UP 1204-0003, ten adult UCD patients were switched from treatment with NaPBA to treatment with HPN-100 at a dosage equivalent to their previous NaPBA dosage with regard to PBA delivery (Lee 2010). In another phase 2 study, HPN-100-005, 11 pediatric UCD patients age six years and up were switched from treatment with NaPBA at a mean dosage of 322 mg/kg/d (range: 198-476 mg/kg/d) to HPN-100 at a PBA equimolar mean dosage of 313 mg/kg/d (range: 192-449 mg/kg/d) (Lichter-Konecki 2011). HPN-100 was found to be at least equivalent to NaPBA in terms of ammonia control. In a third phase 2 study, HPN-100-012, the effect of HPN-100 was evaluated in 15 pediatric UCD patients under six years of age who had previously received NaPBA (Smith 2013). Patients had been receiving a mean daily NaPBA dosage of 5.27 g, and were administered HPN-100 at a dosage that delivered the same amount of PBA. In the HPN-100-012 safety extension, mean ammonia values were found to be within normal limits. Overall, these phase 2 studies established that HPN-100 was at least equal to NaPBA for ammonia control at equivalent dosages.

In the phase 3 study HPN-100-006, the effects of NaPBA and HPN-100 were compared in 45 adult UCD patients who had previously received NaPBA (Diaz 2013). Subjects received a mean dosage of 7.8 g/m²/day (13.95 g/day) of NaPBA or a PBA equivalent mean dosage of 7.55 g/m²/day (13.49 g/day) of HPN-100. HPN-100 was found to be non-inferior to NaPBA for ammonia control. In the phase 3 extended dosing studies HPN-100-007 and HPN-100-005 safety extension, the effect of HPN-100 on ammonia levels was evaluated in 51 adult UCD subjects and 9 pediatric subjects ages 6 and up that had not previously received NaPBA. Subjects received a PBA equivalent mean dosage of 11.84 g/day of HPN-100 (Diaz 2013). Mean ammonia levels were confirmed to be within normal limit.

Data from the four "switchover" studies that compared HPN-100 and NaPBA administration (UP1204-003, HPN-100-005, HPN-100-006, and HPN-100-012) has been used previously to develop a population pharmacokinetic (PK) model that predicted PAA exposure as Cmax and AUC for HPN-100 in UCD populations ranging from 6 to 72 years (Monteleone 2013). In that model, smaller BSA subjects were found to exhibit smaller PK values for clearance, volume, and presystemic conversion than subjects with larger BSA values. As disclosed herein, the data from these same four studies has been further analyzed and combined with data from three additional studies (HPN-100-005 safety extension, HPN-100-0007, and HPN-100-012 safety extension) to develop an updated population PK model that extends to subjects under 6 years of age. This updated model has been used to perform dosing simulations so that PAA exposure can be assessed across various age ranges. These dosing simulations can be used to develop more accurate dosing strategies, including improved dosing strategies for pediatric subjects.

When subjects were broken down by age, dosage levels normalized for BSA for different subgroups of pediatric subjects were found to vary significantly. Pediatric subjects age 6 to 17 years were found to receive a higher normalized dosage of PBA than adult subjects despite receiving a lower actual dosage, and pediatric subjects age 6 to 11 years received a higher normalized dosage than other pediatric subjects and adult subjects. When subjects were broken down by BSA, those below a cutoff value of 1.3 m² were found to receive a higher normalized dosage of PBA than subjects at or above the cutoff. The data suggests that children age 12 to 18 years received a normalized dosage more similar to adults (median of 7.32 g/m²/day vs. 7.13 g/m²/day) than to younger children ages 6 to 11 years children (median of 7.32 g/m²/day vs. 8.98 g/m²/day). BSA was found to be a better differentiator for defining presystematic conversion of HPN-100 or NaPBA to nitrogen scavenging drugs than simply categorizing subjects as adult or pediatric. Dosages for subjects with a BSA below 1.3 m² ranged from 1.09 to 13.97 g/m²/day PBA, with a 25th percentile value of 6.25 g/m²/day, a 75th percentile value of 9.9 g/m²/day, and a median of 8.35 g/m²/day. Dosages for subjects with a BSA of 1.3 m² of greater, on the other hand, ranged from 0.67 to 14.27 g/m²/day, with a 25th percentile value of 5.33 g/m²/day, a 75th percentile value of 8.79 g/m²/day, and a median of 7.18 g/m²/day.

Overall, the findings disclosed herein suggest that PAA prodrug dosage can be more precisely tailored based on BSA. Accordingly, methods are provided herein for more precisely calculating an effective dosage for a subject, particularly a pediatric subject, based on BSA, and for treating UCDs by administering such dosages. The current approved label for HPN-100 recites a dosage of 5 to 12.4 g/m²/day (4.5 to 11.2 mL/m²/day). Using the methods provided herein, HPN-100 may be administered at a more precise dosage within this range based on BSA, allowing for minimization of drug side effects without a decrease in efficacy.

Methods of determining or adjusting an effective dosage of a PAA prodrug in a subject with a UCD based on BSA, and methods of treating a subject with a UCD using the effective and/or adjusted dosage. The invention relates to a method of determining an effective dosage of glyceryl tri-[4-phenylbutyrate], as defined in claim 1. The invention also relates to glyceryl tri-[4-phenylbutyrate] for use in treating a urea cycle disorder, as defined in claim 4. Provided herein are methods of determining an effective dosage of a PAA prodrug for treating a UCD in a subject in need thereof based on the subject's BSA. In these methods, the effective dosage is calculated as a function of BSA, i.e., the effective dosage units incorporate BSA. For example, in certain embodiments the dosage units for the effective dosage are g/m²/day. If the subject's BSA is at or above a predetermined cutoff value, the effective dosage is lower as a function of BSA than if the BSA is below the predetermined cutoff value. In certain embodiments, these methods include a step of calculating a subject's BSA. In other embodiments, the BSA has been previously determined, for example in a previous visit to a medical professional. In certain embodiments, the dosage determination methods provided herein are used to decide between two discrete potential effective dosages, i.e., a lower first potential effective dosage and a higher second potential effective dosage. In other embodiments, the methods are used to decide between two potential effective dosage ranges, i.e., a lower first potential effective dosage range and a higher second potential effective dosage range. Accordingly, the term "effective dosage" as used herein with regard to PAA prodrugs may refer to either a discrete dosage (e.g., 7.18 g/m²/day) or to a dosage range (e.g., 5.33 to 8.79 g/m²/day).

Provided herein are methods of treating UCD in a subject in need thereof that incorporate the dosage determination methods provided herein. These methods comprise administering an effective dosage of PAA prodrug, wherein the effective dosage is determined based on the subject's BSA. If the BSA is at or above a predetermined cutoff value, the effective dosage is lower as a function of BSA than if the BSA is below the predetermined cutoff value.

A "subject" or "subject in need thereof" as used herein with regard to UCD treatment refers to any human subject, adult or pediatric, currently or previously diagnosed with a UCD, deemed at risk of developing a UCD based on one or more genetic or environmental factors, or exhibiting (currently or in the past) one or more symptoms associated with a UCD. A pediatric subject refers to any subject 18 years of age or younger.

The term "about" as used herein means within 10% or within 100 µg/mL of a stated value or a range of values. For example, in certain embodiments, "about" may mean within 10% of a predetermined threshold urinary PAGN level. Therefore, in certain embodiments, "about" may mean within 10%, 9.5%, 9%, 8.5%, 8%, 7.5%, 7%, 6.5%, 6%, 5.5%, 5%, 4.5%, 4%, 3.5%, 3%, 2.5%, 2%, 1.5%, 1%, or 0.5% of a predetermined threshold urinary PAGN level. For example, where the predetermined threshold urinary PAGN level is about 5000 µg/mL, the predetermined threshold urinary PAGN level value may be 4500 µg/mL, 4525 µg/mL, 4550 µg/mL, 4575 µg/mL, 4600 µg/mL, 4625 µg/mL, 4650 µg/mL, 4675 µg/mL, 4700 µg/mL, 4725 µg/mL, 4750 µg/mL, 4775 µg/mL, 4800 µg/mL, 4825 µg/mL, 4850 µg/mL, 4875 µg/mL, 4900 µg/mL, 4925 µg/mL, 4950 µg/mL, 4975 µg/mL, 5025 µg/mL, 5050 µg/mL, 5075 µg/mL, 5100 µg/mL, 5125 µg/mL, 5150 µg/mL, 5175 µg/mL, 5200 µg/mL, 5225 µg/mL, 5250 µg/mL, 5275 µg/mL, 5300 µg/mL, 5325 µg/mL, 5350 µg/mL, 5375 µg/mL, 5400 µg/mL, 5425 µg/mL, 5450 µg/mL, 5475 µg/mL, or 5500 µg/mL. In certain embodiments, the term "about" means within 100 µg/mL of a predetermined threshold urinary PAGN level. In certain embodiments, the predetermined threshold urinary PAGN level may be a value that is at or within 100 µg/mL of the predetermined threshold urinary PAGN level. Therefore, in certain embodiments, the predetermined threshold urinary PAGN level may be a value that is at or within 100 µg/mL, 95 µg/mL, 90 µg/mL, 85 µg/mL, 80 µg/mL, 75 µg/mL, 70 µg/mL, 65 µg/mL, 60 µg/mL, 55 µg/mL, 50 µg/mL, 45 µg/mL, 40 µg/mL, 35 µg/mL, 30 µg/mL, 25 µg/mL, 20 µg/mL, 15 µg/mL, 10 µg/mL, or 5 µg/mL of the predetermined threshold urinary PAGN level. For example, where the predetermined threshold urinary PAGN level is about 5000 µg/mL, the predetermined threshold urinary PAGN level may be 4900 µg/mL, 4905 µg/mL, 4910 µg/mL, 4915 µg/mL, 4920 µg/mL, 4925 µg/mL, 4930 µg/mL, 4935 µg/mL, 4940 µg/mL, 4945 µg/mL, 4950 µg/mL, 4955 µg/mL, 4960 µg/mL, 4965 µg/mL, 4970 µg/mL, 4975 µg/mL, 4980 µg/mL, 4985 µg/mL, 4990 µg/mL, 4995 µg/mL, 5005 µg/mL, 5010 µg/mL, 5015 µg/mL, 5020 µg/mL, 5025 µg/mL, 5030 µg/mL, 5035 µg/mL, 5040 µg/mL, 5045 µg/mL, 5050 µg/mL, 5055 µg/mL, 5060 µg/mL, 5065 µg/mL, 5070 µg/mL, 5075 µg/mL, 5080 µg/mL, 5085 µg/mL, 5090 µg/mL, 5095 µg/mL, or 5100 µg/mL.

The predetermined cutoff value for BSA is 1.3 m². If the subject has a BSA at or above 1.3 m², the effective dosage of PAA prodrug for the subject is lower than if the subject has a BSA below 1.3 m².

In certain embodiments, the methods of determining an effective dosage provided herein comprise calculating a BSA for a subject and determining whether the BSA is at, below, or above 1.3 m². If the BSA is at or above 1.3 m², the effective dosage is a first dosage, and if the BSA is below 1.3 m², the effective dosage is a second dosage. In these embodiments, the second dosage is higher as a function of BSA than the first dosage.

In certain embodiments, the methods of treatment provided herein comprise calculating a BSA for a subject, determining whether the BSA is at, below, or above 1.3 m², and then administering an effective dosage of PAA prodrug to the subject. If the BSA is at or above 1.3 m², the effective dosage is a first dosage, and if the BSA is below 1.3 m², the effective dosage is a second dosage. In these embodiments, the second dosage is higher than the first dosage as a function of BSA.

The effective dosage of glyceryl tri-[4-phenylbutyrate] for a subject with a BSA at or above 1.3 m² is 7.3 to 8.79 g/m²/day. In certain embodiments, the effective dosage is a 8 g/m²/day. The effective dosage of glyceryl tri-[4-phenylbutyrate] for a subject with a BSA below 1.3 m² is 9 to 9.9 g/m²/day. In certain embodiments, the effective dosage is 9 to 9.5 g/m²/day

In certain embodiments, the methods of dosage determination provided herein comprise calculating a BSA for a subject and determining an effective dosage of PAA prodrug based on whether the BSA is at, below, or above 1.3 m², where the effective dosage is a first dosage if the BSA is at or above 1.3 m² or a second dosage if the BSA is below 1.3 m², and where first dosage is about 7.3 to 8.79 g/m²/day and the second dosage is about 9 to 9.9 g/m²/day. In certain of these embodiments, the first dosage is about 8 g/m²/day, and the second dosage is about 9 to 9.5 g/m²/day.

In certain embodiments, the methods of treatment provided herein comprise calculating a BSA for a subject, determining whether the BSA is at, below, or above 1.3 m², and then administering a first dosage of a PAA prodrug if the BSA is at or above 1.3 m² or a second dosage if the BSA is below 1.3 m², where the first dosage is about 7.3 to 8.79 g/m²/day, and the second dosage is about 9 to 9.9 g/m²/day. In certain of these embodiments, the first dosage is about 8 g/m²/day, and the second dosage is about 9 to 9.5 g/m²/day.

In certain embodiments, the methods of treatment provided herein comprise administering an effective dosage of a PAA prodrug to a subject in need thereof with a BSA of 1.3 m² or greater, wherein the effective dosage of the PAA prodrug is about 7.3 to 8.79 g/m²/day. In certain embodiments, the effective dosage for a subject with a BSA at or above 1.3 m² is about 8 g/m²/day In still other embodiments, the subject may be a pediatric subject under the age of 12.

In certain embodiments, the methods of treatment provided herein comprise administering an effective dosage of a PAA prodrug to a subject in need thereof with a BSA of below 1.3 m² by administering an effective dosage of a PAA prodrug, where the effective dosage of the PAA prodrug is about 9 to 9.9 g/m²/day. In certain embodiments, the effective dosage is about 9 to 9.5 g/m²/day. In certain embodiments, the subject is a pediatric subject under the age of 12.

A UCD as used herein refers to any subtype of UCD, including but not limited to carbamylphosphate synthetase deficiency, ornithine transcarbamylase deficiency, argininosuccinic acid synthetase deficiency, arginosuccinic acid lyase deficiency, arginase deficiency, hyperornithinemia-hyperammonemia-homocitrullinemia (HHH) syndrome, and citrin deficiency (citrullinemia type II).

A "PAA prodrug" as used herein refers to any drug that contains or is metabolized following administration to PAA. In certain embodiments of the methods provided herein, the PAA prodrug may be HPN-100, PBA, a pharmaceutically salt of PBA such as NaPBA, or a pharmaceutically acceptable ester, acid, or derivative of any of these compounds. In certain embodiments, the PAA prodrug is orally administrable. In other embodiments, the PAA prodrug may be administered parenterally. The PAA prodrug used in the invention is HPN-100.

In certain embodiments of the methods of treatment provided herein, the subject may be administered an effective dosage of PAA prodrug only once. In other embodiments, the effective dosage of the PAA prodrug may be administered multiple times, for example until a specific therapeutic benchmark is reached. In these embodiments, the PAA prodrug may be administered multiple times per day, once per day, once every 2-6 days, once per week, once every 2-4 weeks, or once a month. In certain of these embodiments, the PAA prodrug is administered for a period long enough to reach steady state. In certain embodiments, the subject being treated has previously been administered a PAA prodrug or another nitrogen scavenging drug. In certain of these embodiments, the effective dosage administered to the subject may be the same or different than a previous dosage administered to the subject. In other embodiments, the subject has not been treated previously with a PAA prodrug or another nitrogen scavenging drug.

In certain embodiments, the effective dosage of PAA prodrug may be varied over time based on the subject's response. For example, where the effective dosage is a range, a subject who is administered a dosage at the lower end of the range may later be administered a higher dosage within the range if the original dosage does not generate a sufficient therapeutic response. Similarly, the dosage may be decreased to the lower end of the range where the therapeutic response has been deemed effective, and in certain of these embodiments the dosage may be steadily lowered over time until administered is ceased.

Certain embodiments of the dosage determination and treatment methods disclosed herein may take into account other factors in addition to BSA when determining an effective dosage of a PAA prodrug. For example, the subject's age or overall health or the severity of the subject's UCD may be taken into account. In certain embodiments where the effective dosage is a range, a subject exhibiting a more severe UCD may be administered a dosage closer to the upper limit of the range than a subject exhibiting a less severe UCD. Other factors that may be taken into account when determining an effective dosage of a PAA prodrug include diet (e.g., protein intake), endogenous waste nitrogen capacity (e.g., urea synthesis capacity), the ratio of fasting blood ammonia levels to an upper limit of normal (see, e.g., US Patent No. 8,404,215), or the ratio of PAA to PAGN (see, e.g., US Patent Publ. No. 2013/0281530). Another factor that may be taken into account when determining the effective dosage of a PAA prodrug is the percent conversion of PAA prodrug to urinary PAGN. The mean conversion percentage of PAA prodrug to urinary PAGN has previously been reported as about 60 to 75% in subjects with UCD or HE (see US Patent Publ. No. 2010/0008859). Accordingly, in certain
embodiments the methods of dosage determination and treatment provided herein take into account this percent conversion of PAA prodrug to urinary PAGN. For example, in certain embodiments the methods include a step of determining a target urinary PAGN output based on target nitrogen removal, and calculating PAA prodrug dosage based on a mean percent conversion of 60 to 75%, 60 to 65%, or about 60%.

In certain embodiments, the methods of dosage determination, treatment, and compliance evaluation disclosed herein may be incorporated into a larger dosage determination protocol that takes into account multiple factors. An example of such a protocol is set forth in Figure 5.

As set forth in the protocol of Figure 5, a subject is initially evaluated for fasting ammonia level, urinary PAGN level, and/or PAA:PAGN ratio. Evaluation of BSA may be incorporated into this initial step. In certain embodiments, the subject has previously received one or more PAA prodrugs. In other embodiments, the subject has not yet received a PAA prodrug.

If the subject's fasting ammonia level is high, the subject is evaluated for urinary PAGN levels based on the compliance methods disclosed herein. In certain embodiments, fasting ammonia level is classified as high if it is greater than or equal to half the upper limit of normal. Fasting ammonia levels less than half the upper limit of normal have been reported to increase the likelihood that the average daily ammonia will be within normal limits and a decreased risk and frequency of hyperammonemic crises (Lee 2013). In other embodiments, fasting ammonia level is classified as high if it is at or above a predetermined threshold value.

If the subject's urinary PAGN level is below a predetermined threshold value (e.g., below the 25th percentile value for the subject's dosage group), the subject may undergo additional compliance evaluation. If the urinary PAGN level is above the predetermined threshold value, PAA:PAGN ratio is evaluated.

If the PAA:PAGN ratio is below 2.5, the dosage of PAA prodrug may be increased. If the ratio is at or above 2.5, the frequency of administration may be increased. PAA:PAGN ratio is clinically useful in that it represents an inherent measure of the efficiency with which PAA is converted to PAGN in an individual subject. A ratio greater than 2.5 (where both PAA and PAGN are expressed in µg/mL) is associated with probabilities of PAA levels exceeding 400 µg/mL ranging from approximately 25% to 36%, whereas a ratio less than or equal to 2.5 is associated with an approximately 1% risk of a high PAA value. Thus, a ratio greater than 2.5 with unexplained neurological adverse events and normal ammonia provides a clue that cautious changes to dose or dosing regimen should be considered. A ratio at or below 2.5 indicates efficient conversion of PAA to PAGN and suggests that the dosage of PAA prodrug can be increased if necessary (Mokhtarani 2013).

If the fasting ammonia level in the initial step of the evaluation is normal, one or more symptoms may be evaluated. In certain embodiments, fasting ammonia level is classified as normal if it is less than half the upper limit of normal. In other embodiments, fasting ammonia level is classified as normal if it below a predetermined threshold value. If the symptoms are within acceptable limits or have showed a desired decrease, the subject may continue at their current dosage or receive a decreased dosage. If the symptoms are above acceptable limits or have showed no change or an increase, the subject's PAA:PAGN ratio is evaluated. If the ratio is less than 2.5, the subject may be evaluated for other causes of lack of symptom cessation. If the ratio is at or above 2.5, the subject may be administered a reduced dosage and/or an increased frequency of dosage. All or part of this protocol may be repeated at various intervals. These intervals may be predetermined (e.g., once every week), or they may be variable depending on various factors such as symptom severity.

Methods of evaluating compliance with a PAA prodrug treatment regimen and treating a subject with a UCD based on BSA. Provided herein in certain embodiments are methods of evaluating compliance with a PAA prodrug treatment regimen in a subject with a UCD based on BSA as shown in Example 4. In certain embodiments, the method of evaluating compliance comprises classifying the subject into a BSA group based on the subject's BSA, determining a urinary PAGN level for the subject, and comparing the urinary PAGN level to a predetermined threshold urinary PAGN level. In certain embodiments, a urinary PAGN level below the predetermined threshold urinary PAGN level indicates that the subject is non-compliant with the PAA prodrug treatment regimen. In certain embodiments, a urinary PAGN level at or above the predetermined threshold urinary PAGN level indicates that the subject is compliant with the PAA prodrug treatment regimen. In certain embodiments, a dosage of PAA prodrug may be administered to the subject if the urinary PAGN level for the subject is below the predetermined threshold urinary PAGN level for the subject's BSA group. In certain embodiments, the dosage of PAA prodrug may be an effective dosage of a PAA prodrug as described herein.

Also provided herein in certain embodiments are methods of treating a UCD in a subject in need thereof based on the subject's BSA as shown in Example 4. In certain embodiments, the method of treating a UCD in a subject in need thereof comprises classifying the subject into a BSA group based on the subject's BSA, determining a urinary PAGN level for the subject, comparing the urinary PAGN level to a predetermined threshold urinary PAGN level, and administering a dosage of PAA prodrug if the urinary PAGN level for the subject is below the predetermined threshold urinary PAGN level for the subject's BSA group. In certain embodiments, the dosage of PAA prodrug may be an effective dosage of a PAA prodrug as described herein.

In certain embodiments, the subject's BSA may be calculated. In other embodiments, the BSA has been previously determined, for example in a previous visit to a medical professional. In certain embodiments, the subject is classified into a BSA group based on the subject's BSA. In certain embodiments, the BSA group may be one of two groups: (1) a BSA of less than or equal to 1.3 m² or (2) a BSA greater than 1.3 m². In certain embodiments, the method further comprises a step of determining a urinary PAGN level for the subject. In certain embodiments, a urine sample may be obtained from the subject and urinary PAGN levels for the subject may be measured. In certain embodiments, the urine sample may be a spot urine sample obtained from the subject prior to the first drug administration and/or meal of the day. In certain embodiments, the urine sample may be a spot urine sample obtained from the first void of morning urine sample. In certain embodiments, the method further comprises a step of comparing the subject's urinary PAGN level to a predetermined threshold urinary PAGN level. As provided in Table 5, the predetermined threshold urinary PAGN level may be the urinary PAGN level of the 5th percentile, 10th percentile, 25th percentile, 50th percentile, 75th percentile, 90th percentile, or 95th percentile of the subject's BSA group. In certain embodiments, the predetermined threshold urinary PAGN level value may be rounded to the nearest 1000 µg/mL. In certain embodiments, the value of the predetermined threshold urinary PAGN level is specific to the subject's BSA group. In certain embodiments, a subject may be classified as non-compliant or potentially non-compliant if the subject's urinary PAGN level is below a predetermined threshold level for the subject's specific BSA group. In certain embodiments, a subject may be classified as compliant if the subject's urinary PAGN level is at or above a predetermined threshold level for their specific BSA group. In certain embodiments, a subject classified as non-compliant or potentially non-compliant is subjected to additional evaluations to assess compliance. In certain embodiments, the subject may be administered PAA prodrug under more tightly monitored conditions, for example under direct supervision of a medical professional. In certain embodiments, a dosage of PAA prodrug may be administered to the subject if the urinary PAGN level for the subject is below the predetermined threshold urinary PAGN level for their BSA group. In certain embodiments, the dosage of PAA prodrug may be an effective dosage of a PAA prodrug as described herein. It is recommended to assess compliance and/or method of drug administration if the urinary PAGN concentrations from first void of morning urine samples are below the predetermined threshold urinary PAGN level.

*5th percentile urinary PAGN level:* In certain embodiments, the predetermined threshold urinary PAGN level may be the 5th percentile urinary PAGN level for the subject's BSA group as provided in Table 5. In certain embodiments, the predetermined threshold urinary PAGN level value for a subject in the less than or equal to 1.3 m² BSA group may be about 1062 µg/mL, which may be rounded to the nearest 1000 µg/mL, i.e., 1000 µg/mL. In certain embodiments, a subject in in the less than or equal to 1.3 m² group may be classified as non-compliant or potentially non-compliant if they exhibit a urinary PAGN level below about 1062 µg/mL or below about 1000 µ/mL. In certain embodiments, the subject may be classified as compliant if they exhibit a urinary PAGN level at or above about 1062 µg/mL or at or above about 1000 µg/mL. In certain embodiments, the predetermined threshold urinary PAGN level value for a subject in the greater than 1.3 m² group may be about 3379 µg/mL, which may be rounded to the nearest 1000 µg/mL, i.e., 3000 µg/mL. In certain embodiments, a subject in the greater than 1.3 m² group may be classified as non-compliant or potentially non-compliant if they exhibit a urinary PAGN level below about 3379 µg/mL or below about 3000 µg/mL. In certain embodiments, the subject may be classified as compliant if they exhibit a urinary PAGN level at or above about 3379 µg/mL or at or above about 3000 µg/mL. In certain embodiments, a dosage of PAA prodrug may be administered to the subject if the urinary PAGN level for the subject is below the predetermined threshold urinary PAGN level for their BSA group. In certain embodiments, the dosage of PAA prodrug may be an effective dosage of a PAA prodrug as described herein.

*10th percentile urinary PAGN level:* In certain embodiments, the predetermined threshold urinary PAGN level may be the 10th percentile urinary PAGN level for the subject's BSA group as provided in Table 5. In certain embodiments, the predetermined threshold urinary PAGN level value for a subject in the less than or equal to 1.3 m² BSA group may be about 3646 µg/mL, which may be rounded to the nearest 1000 µg/mL, i.e., 4000 µg/mL. In certain embodiments, a subject in the less than or equal to 1.3 m² BSA group may be classified as non-compliant or potentially non-compliant if they exhibit a urinary PAGN level below about 3646 µg/mL or below about 4000 µg/mL. In certain embodiments, the subject may be classified as compliant if they exhibit a urinary PAGN level at or above about 3646 µg/mL or at or above about 4000 µg/mL. In certain embodiments, the predetermined threshold urinary PAGN level value for a subject in the greater than 1.3 m² BSA group may be about 4079 µg/mL, which may be rounded to the nearest 1000 µg/mL, i.e., 4000 µg/mL. In certain embodiments, a subject in the greater than 1.3 m² BSA group may be classified as non-compliant or potentially non-compliant if they exhibit a urinary PAGN level below about 4079 µg/mL or below about 4000 µg/mL. In certain embodiments, the subject may be classified as compliant if they exhibit a urinary PAGN level at or above about 4079 µg/mL or at or above about 4000 µg/mL. In certain embodiments, a dosage of PAA prodrug may be administered to the subject if the urinary PAGN level for the subject is below the predetermined threshold urinary PAGN level for their BSA group. In certain embodiments, the dosage of PAA prodrug may be an effective dosage of a PAA prodrug as described herein.

*25th percentile urinary PAGN level:* In certain preferred embodiments, the predetermined threshold urinary PAGN level may be the 25th percentile urinary PAGN level for the subject's BSA group as provided in Table 5. In certain embodiments, the predetermined threshold urinary PAGN level value for a subject in the less than or equal to 1.3 m² BSA group may be about 8390 µg/mL, which may be rounded to the nearest 1000 µg/mL, i.e., 8000 µg/mL. In certain embodiments, a subject in the less than or equal to 1.3 m² BSA group may be classified as non-compliant or potentially non-compliant if they exhibit a urinary PAGN level below about 8390 µg/mL or below about 8000 µg/mL. In certain embodiments, the subject may be classified as compliant if they exhibit a urinary PAGN level at or above about 8390 µg/mL or at or above about 8000 µg/mL. In certain embodiments, the predetermined threshold urinary PAGN level value for a subject in the greater than 1.3 m² BSA group may be about 5259 µg/mL, which may be rounded to the nearest 1000 µg/mL, i.e., 5000 µg/mL. In certain embodiments, a subject in the greater than 1.3 m² BSA group may be classified as non-compliant or potentially non-compliant if they exhibit a urinary PAGN level below about 5259 µg/mL or below about 5000 µg/mL. In certain embodiments, the subject may be classified as compliant if they exhibit a urinary PAGN level at or above about 5259 µg/mL or at or above about 5000 µg/mL. In certain embodiments, a dosage of PAA prodrug may be administered to the subject if the urinary PAGN level for the subject is below the predetermined threshold urinary PAGN level for their BSA group. In certain embodiments, the dosage of PAA prodrug may be an effective dosage of a PAA prodrug as described herein.

*50th percentile urinary PAGN level:* In certain embodiments, the predetermined threshold urinary PAGN level may be the 50th percentile urinary PAGN level for the subject's BSA group as provided in Table 5. In certain embodiments, the predetermined threshold urinary PAGN level value for a subject in the less than or equal to 1.3 m² BSA group may be about 17075 µg/mL, which may be rounded to the nearest 1000 µg/mL, i.e., 17000 µg/mL. In certain embodiments, a subject in the less than or equal to 1.3 m² BSA group may be classified as non-compliant or potentially non-compliant if they exhibit a urinary PAGN level below about 17075 µg/mL or below about 17000 µg/mL. In certain embodiments, the subject may be classified as compliant if they exhibit a urinary PAGN level at or above about 17075 µg/mL or at or above about 17000 µg/mL. In certain embodiments, the predetermined threshold urinary PAGN level value for a subject in the greater than 1.3 m² BSA group may be about 7749 µg/mL, which may be rounded to the nearest 1000 µg/mL, i.e., 8000 µg/mL. In certain embodiments, a subject in the greater than 1.3 m² BSA group may be classified as non-compliant or potentially non-compliant if they exhibit a urinary PAGN level below about 7749 µg/mL or below about 8000 µg/mL. In certain embodiments, the subject may be classified as compliant if they exhibit a urinary PAGN level at or above about 7749 µg/mL or at or above about 8000 µg/mL. In certain embodiments, a dosage of PAA prodrug may be administered to the subject if the urinary PAGN level for the subject is below the predetermined threshold urinary PAGN level for their BSA group. In certain embodiments, the dosage of PAA prodrug may be an effective dosage of a PAA prodrug as described herein.

*75th percentile urinary PAGN level:* In certain embodiments, the predetermined threshold urinary PAGN level may be the 75th percentile urinary PAGN level for the subject's BSA group as provided in Table 5. In certain embodiments, the predetermined threshold urinary PAGN level value for a subject in the less than or equal to 1.3 m² BSA group may be about 25466 µg/mL, which may be rounded to the nearest 1000 µg/mL, i.e., 25000 µg/mL. In certain embodiments, a subject in the less than or equal to 1.3 m² BSA group may be classified as non-compliant or potentially non-compliant if they exhibit a urinary PAGN level below about 25466 µg/mL or below about 25000 µg/mL. In certain embodiments, the subject may be classified as compliant if they exhibit a urinary PAGN level at or above about 25466 µg/mL or at or above about 25000 µg/mL. In certain embodiments, the predetermined threshold urinary PAGN level value for a subject in the greater than 1.3 m² BSA group may be about 11916 µg/mL, which may be rounded to the nearest 1000 µg/mL, i.e., 12000 µg/mL. In certain embodiments, a subject in the greater than 1.3 m² BSA group may be classified as non-compliant or potentially non-compliant if they exhibit a urinary PAGN level below about 11916 µg/mL or below about 12000 µg/mL. In certain embodiments, the subject may be classified as compliant if they exhibit a urinary PAGN level at or above about 11916 µg/mL or at or above about 12000 µg/mL. In certain embodiments, a dosage of PAA prodrug may be administered to the subject if the urinary PAGN level for the subject is below the predetermined threshold urinary PAGN level for their BSA group. In certain embodiments, the dosage of PAA prodrug may be an effective dosage of a PAA prodrug as described herein.

*90th percentile urinary PAGN level:* In certain embodiments, the predetermined threshold urinary PAGN level may be the 90th percentile urinary PAGN level for the subject's BSA group as provided in Table 5. In certain embodiments, the predetermined threshold urinary PAGN level value for a subject in the less than or equal to 1.3 m² BSA group may be about 30830 µg/mL, which may be rounded to the nearest 1000 µg/mL, i.e., 31000 µg/mL. In certain embodiments, a subject in the less than or equal to 1.3 m² BSA group may be classified as non-compliant or potentially non-compliant if they exhibit a urinary PAGN level below about 30830 µg/mL or below about 31000 µg/mL. In certain embodiments, the subject may be classified as compliant if they exhibit a urinary PAGN level at or above about 30830 µg/mL or at or above about 31000 µg/mL. In certain embodiments, the predetermined threshold urinary PAGN level value for a subject in the greater than 1.3 m² BSA group may be about 15993 µg/mL, which may be rounded to the nearest 1000 µg/mL, i.e., 16000 µg/mL. In certain embodiments, a subject in the greater than 1.3 m² BSA group may be classified as non-compliant or potentially non-compliant if they exhibit a urinary PAGN level below about 15993 µg/mL or below about 16000 µg/mL. In certain embodiments, the subject may be classified as compliant if they exhibit a urinary PAGN level at or above about 15993 µg/mL or at or above about 16000 µg/mL. In certain embodiments, a dosage of PAA prodrug may be administered to the subject if the urinary PAGN level for the subject is below the predetermined threshold urinary PAGN level for their BSA group. In certain embodiments, the dosage of PAA prodrug may be an effective dosage of a PAA prodrug as described herein.

*95th percentile urinary PAGN level:* In certain embodiments, the predetermined threshold urinary PAGN level may be the 95th percentile urinary PAGN level for the subject's BSA group as provided in Table 5. In certain embodiments, the predetermined threshold urinary PAGN level value for a subject in the less than or equal to 1.3 m² BSA group may be about 35516 µg/mL, which may be rounded to the nearest 1000 µg/mL, i.e., 36000 µg/mL. In certain embodiments, a subject in the less than or equal to 1.3 m² BSA group may be classified as non-compliant or potentially non-compliant if they exhibit a urinary PAGN level below about 35516 µg/mL or below about 36000 µg/mL. In certain embodiments, the subject may be classified as compliant if they exhibit a urinary PAGN level at or above about 35516 µg/mL or at or above about 36000 µg/mL. In certain embodiments, the predetermined threshold urinary PAGN level value for a subject in the greater than 1.3 m² BSA group may be about 20320 µg/mL, which may be rounded to the nearest 1000 µg/mL, i.e., 20000 µg/mL. In certain embodiments, a subject in the greater than 1.3 m² BSA group may be classified as non-compliant or potentially non-compliant if they exhibit a urinary PAGN level below about 20320 µg/mL or below about 20000 µg/mL. In certain embodiments, the subject may be classified as compliant if they exhibit a urinary PAGN level at or above about 20320 µg/mL or at or above about 20000 µg/mL. In certain embodiments, a dosage of PAA prodrug may be administered to the subject if the urinary PAGN level for the subject is below the predetermined threshold urinary PAGN level for their BSA group. In certain embodiments, the dosage of PAA prodrug may be an effective dosage of a PAA prodrug as described herein.

Methods of evaluating compliance with a PAA prodrug treatment regimen and treating a subject 2 years of age or older with a UCD based on BSA. Provided herein in certain embodiments are methods of evaluating compliance with a PAA prodrug treatment regimen in a subject with a UCD who is 2 years of age or older based on BSA as shown in Example 5. In certain embodiments, the method of evaluating compliance comprises classifying the subject into a BSA group based on the subject's BSA, determining a urinary PAGN level for the subject, and comparing the urinary PAGN level to a predetermined threshold urinary PAGN level. In certain embodiments, a urinary PAGN level below the predetermined threshold urinary PAGN level indicates that the subject is non-compliant with the PAA prodrug treatment regimen. In certain embodiments, a urinary PAGN level at or above the predetermined threshold urinary PAGN level indicates that the subject is compliant with the PAA prodrug treatment regimen. In certain embodiments, a dosage of PAA prodrug may be administered to the subject if the urinary PAGN level for the subject is below the predetermined threshold urinary PAGN level for the subject's BSA group. In certain embodiments, the dosage of PAA prodrug may be an effective dosage of a PAA prodrug as described herein.

Also provided herein in certain embodiments are methods of treating a UCD in a subject in need thereof who is 2 years of age or older based on the subject's BSA as shown in Example 5. In certain embodiments, the method of treating a UCD in a subject in need thereof comprises classifying the subject into a BSA group based on the subject's BSA, determining a urinary PAGN level for the subject, comparing the urinary PAGN level to a predetermined threshold urinary PAGN level, and administering a dosage of PAA prodrug if the urinary PAGN level for the subject is below the predetermined threshold urinary PAGN level for the subject's BSA group. In certain embodiments, the dosage of PAA prodrug may be an effective dosage of a PAA prodrug as described herein.

In certain embodiments, the subject's BSA may be calculated. In other embodiments, the BSA has been previously determined, for example in a previous visit to a medical professional. In certain embodiments, the subject who is 2 years of age or older is classified into a BSA group based on the subject's BSA. In certain embodiments, the BSA group may be one of two groups: (1) a BSA of less than or equal to 1.3 m² or (2) a BSA greater than 1.3 m². In certain embodiments, the method further comprises a step of determining a urinary PAGN level for the subject. In certain embodiments, a urine sample may be obtained from the subject and urinary PAGN levels for the subject may be measured. In certain embodiments, the urine sample may be a spot urine sample obtained from the subject prior to the first drug administration and/or meal of the day. In certain embodiments, the urine sample may be a spot urine sample obtained from the first void of morning urine sample. In certain embodiments, the method further comprises a step of comparing the subject's urinary PAGN level to a predetermined threshold urinary PAGN level. As provided in Table 6, the predetermined threshold urinary PAGN level may be the urinary PAGN level of the 5th percentile, 10th percentile, 25th percentile, 75th percentile, 90th percentile, or 95th percentile of the subject's BSA group. In certain embodiments, the predetermined threshold urinary PAGN level value may be rounded to the nearest 1000 µg/mL. In certain embodiments, the value of the predetermined threshold urinary PAGN level is specific to the subject's BSA group. In certain embodiments, a subject may be classified as non-compliant if the subject's urinary PAGN level is below a predetermined threshold level for the subject's specific BSA group. In certain embodiments, a subject may be classified as potentially non-compliant if the subject's urinary PAGN level is below a predetermined threshold level for the subject's specific BSA group. In certain embodiments, a subject may be classified as compliant if the subject's urinary PAGN level is at or above a predetermined threshold level for their specific BSA group. In certain embodiments, a subject classified as non-compliant or potentially non-compliant is subjected to additional evaluations to assess compliance. In certain embodiments, the subject may be administered PAA prodrug under more tightly monitored conditions, for example under direct supervision of a medical professional. In certain embodiments, a dosage of PAA prodrug may be administered to the subject if the urinary PAGN level for the subject is below the predetermined threshold urinary PAGN level for their BSA group. In certain embodiments, the dosage of PAA prodrug may be an effective dosage of a PAA prodrug as described herein. It is recommended to assess compliance and/or method of drug administration if the urinary PAGN concentrations from first void of morning urine samples are below the predetermined threshold urinary PAGN level.

*5th percentile urinary PAGN level:* In certain embodiments, the predetermined threshold urinary PAGN level may be the 5th percentile urinary PAGN level for the subject's BSA group as provided in Table 6. In certain embodiments, the predetermined threshold urinary PAGN level value for a subject who is 2 years of age or older in the less than or equal to 1.3 m² BSA group may be about 622 µg/mL, which may be rounded to the nearest 1000 µg/mL, i.e., 1000 µg/mL. In certain embodiments, a subject who is 2 years of age or older in the less than or equal to 1.3 m² group may be classified as non-compliant or potentially non-compliant if they exhibit a urinary PAGN level below about 622 µg/mL or below about 1000 µg/mL. In certain embodiments, the subject who is 2 years of age or older may be classified as compliant if they exhibit a urinary PAGN level at or above about 622 µg/mL or at or above about 1000 µg/mL. In certain embodiments, the predetermined threshold urinary PAGN level value for a subject who is 2 years of age or older in the greater than 1.3 m² group may be about 3379 µg/mL, which may be rounded to the nearest 1000 µg/mL, i.e., 3000 µg/mL. In certain embodiments, a subject who is 2 years of age or older in the greater than 1.3 m² group may be classified as non-compliant or potentially non-compliant if they exhibit a urinary PAGN level below about 3379 µg/mL or below about 3000 µg/mL. In certain embodiments, the subject who is 2 years of age or older may be classified as compliant if they exhibit a urinary PAGN level at or above about 3379 µg/mL or at or above about 3000 µg/mL. In certain embodiments, a dosage of PAA prodrug may be administered to the subject who is 2 years of age or older if the urinary PAGN level for the subject is below the predetermined threshold urinary PAGN level for their BSA group. In certain embodiments, the dosage of PAA prodrug may be an effective dosage of a PAA prodrug as described herein.

*10th percentile urinary PAGN level:* In certain embodiments, the predetermined threshold urinary PAGN level may be the 10th percentile urinary PAGN level for the subject's BSA group as provided in Table 6. In certain embodiments, the predetermined threshold urinary PAGN level value for a subject who is 2 years of age or older in the less than or equal to 1.3 m² BSA group may be about 3479 µg/mL, which may be rounded to the nearest 1000 µg/mL, i.e., 3000 µg/mL. In certain embodiments, a subject who is 2 years of age or older in the less than or equal to 1.3 m² BSA group may be classified as non-compliant or potentially non-compliant if they exhibit a urinary PAGN level below about 3479 µg/mL or below about 3000 µg/mL. In certain embodiments, the subject who is 2 years of age or older may be classified as compliant if they exhibit a urinary PAGN level at or above about 3479 µg/mL or at or above about 3000 µg/mL. In certain embodiments, the predetermined threshold urinary PAGN level value for a subject who is 2 years of age or older in the greater than 1.3 m² BSA group may be about 4079 µg/mL, which may be rounded to the nearest 1000 µg/mL, i.e., 4000 µg/mL. In certain embodiments, a subject who is 2 years of age or older in the greater than 1.3 m² BSA group may be classified as non-compliant or potentially non-compliant if they exhibit a urinary PAGN level below about 4079 µg/mL or below about 4000 µg/mL. In certain embodiments, the subject who is 2 years of age or older may be classified as compliant if they exhibit a urinary PAGN level at or above about 4079 µg/mL or at or above about 4000 µg/mL. In certain embodiments, a dosage of PAA prodrug may be administered to the subject who is 2 years of age or older if the urinary PAGN level for the subject is below the predetermined threshold urinary PAGN level for their BSA group. In certain embodiments, the dosage of PAA prodrug may be an effective dosage of a PAA prodrug as described herein.

*25th percentile urinary PAGN level:* In certain preferred embodiments, the predetermined threshold urinary PAGN level may be the 25th percentile urinary PAGN level for the subject's BSA group as provided in Table 6. In certain embodiments, the predetermined threshold urinary PAGN level value for a subject who is 2 years of age or older in the less than or equal to 1.3 m² BSA group may be about 7412 µg/mL, which may be rounded to the nearest 1000 µg/mL, i.e., 7000 µg/mL. In certain embodiments, a subject who is 2 years of age or older in the less than or equal to 1.3 m² BSA group may be classified as non-compliant or potentially non-compliant if they exhibit a urinary PAGN level below about 7412 µg/mL or below about 7000 µg/mL. In certain embodiments, the subject who is 2 years of age or older may be classified as compliant if they exhibit a urinary PAGN level at or above about 7412 µg/mL or at or above about 7000 µg/mL. In certain embodiments, the predetermined threshold urinary PAGN level value for a subject who is 2 years of age or older in the greater than 1.3 m² BSA group may be about 5259 µg/mL, which may be rounded to the nearest 1000 µg/mL, i.e., 5000 µg/mL. In certain embodiments, a subject who is 2 years of age or older in the greater than 1.3 m² BSA group may be classified as non-compliant or potentially non-compliant if they exhibit a urinary PAGN level below about 5259 µg/mL or below about 5000 µg/mL. In certain embodiments, the subject who is 2 years of age or older in the greater than 1.3 m² BSA group may be classified as compliant if they exhibit a urinary PAGN level at or above about 5259 µg/mL or at or above about 5000 µg/mL. In certain embodiments, a dosage of PAA prodrug may be administered to the subject who is 2 years of age or older if the urinary PAGN level for the subject is below the predetermined threshold urinary PAGN level for their BSA group. In certain embodiments, the dosage of PAA prodrug may be an effective dosage of a PAA prodrug as described herein.

*75th percentile urinary PAGN level:* In certain embodiments, the predetermined threshold urinary PAGN level may be the 75th percentile urinary PAGN level for the subject's BSA group as provided in Table 6. In certain embodiments, the predetermined threshold urinary PAGN level value for a subject who is 2 years of age or older in the less than or equal to 1.3 m² BSA group may be about 23635 µg/mL, which may be rounded to the nearest 1000 µg/mL, i.e., 24000 µg/mL. In certain embodiments, a subject who is 2 years of age or older in the less than or equal to 1.3 m² BSA group may be classified as non-compliant or potentially non-compliant if they exhibit a urinary PAGN level below about 23635 µg/mL or below about 24000 µg/mL. In certain embodiments, the subject may be classified as compliant if they exhibit a urinary PAGN level at or above about 23635 µg/mL or at or above about 24000 µg/mL. In certain embodiments, the predetermined threshold urinary PAGN level value for a subject who is 2 years of age or older in the greater than 1.3 m² BSA group may be about 11916 µg/mL, which may be rounded to the nearest 1000 µg/mL, i.e., 12000 µg/mL. In certain embodiments, a subject who is 2 years of age or older in the greater than 1.3 m² BSA group may be classified as non-compliant or potentially non-compliant if they exhibit a urinary PAGN level below about 11916 µg/mL or below about 12000 µg/mL. In certain embodiments, the subject may be classified as compliant if they exhibit a urinary PAGN level at or above about 11916 µg/mL or at or above about 12000 µg/mL. In certain embodiments, a dosage of PAA prodrug may be administered to the subject who is 2 years of age or older if the urinary PAGN level for the subject is below the predetermined threshold urinary PAGN level for their BSA group. In certain embodiments, the dosage of PAA prodrug may be an effective dosage of a PAA prodrug as described herein.

*90th percentile urinary PAGN level:* In certain embodiments, the predetermined threshold urinary PAGN level may be the 90th percentile urinary PAGN level for the subject's BSA group as provided in Table 6. In certain embodiments, the predetermined threshold urinary PAGN level value for a subject who is 2 years of age or older in the less than or equal to 1.3 m² BSA group may be about 29835 µg/mL, which may be rounded to the nearest 1000 µg/mL, i.e., 30000 µg/mL. In certain embodiments, a subject who is 2 years of age or older in the less than or equal to 1.3 m² BSA group may be classified as non-compliant or potentially non-compliant if they exhibit a urinary PAGN level below about 29835 µg/mL or below about 30000 µg/mL. In certain embodiments, the subject may be classified as compliant if they exhibit a urinary PAGN level at or above about 29835 µg/mL or at or above about 30000 µg/mL. In certain embodiments, the predetermined threshold urinary PAGN level value for a subject who is 2 years of age or older in the greater than 1.3 m² BSA group may be about 15993 µg/mL, which may be rounded to the nearest 1000 µg/mL, i.e., 16000 µg/mL. In certain embodiments, a subject who is 2 years of age or older in the greater than 1.3 m² BSA group may be classified as non-compliant or potentially non-compliant if they exhibit a urinary PAGN level below about 15993 µg/mL or below about 16000 µg/mL. In certain embodiments, the subject may be classified as compliant if they exhibit a urinary PAGN level at or above about 15993 µg/mL or at or above about 16000 µg/mL. In certain embodiments, a dosage of PAA prodrug may be administered to the subject who is 2 years of age or older if the urinary PAGN level for the subject is below the predetermined threshold urinary PAGN level for their BSA group. In certain embodiments, the dosage of PAA prodrug may be an effective dosage of a PAA prodrug as described herein.

*95th percentile urinary PAGN level:* In certain embodiments, the predetermined threshold urinary PAGN level may be the 95th percentile urinary PAGN level for the subject's BSA group as provided in Table 6. In certain embodiments, the predetermined threshold urinary PAGN level value for a subject who is 2 years of age or older in the less than or equal to 1.3 m² BSA group may be about 33944 µg/mL, which may be rounded to the nearest 1000 µg/mL, i.e., 34000 µg/mL. In certain embodiments, a subject who is 2 years of age or older in the less than or equal to 1.3 m² BSA group may be classified as non-compliant or potentially non-compliant if they exhibit a urinary PAGN level below about 33944 µg/mL or below about 34000 µg/mL. In certain embodiments, the subject may be classified as compliant if they exhibit a urinary PAGN level at or above about 33944 µg/mL or at or above about 34000 µg/mL. In certain embodiments, the predetermined threshold urinary PAGN level value for a subject who is 2 years of age or older in the greater than 1.3 m² BSA group may be about 20320 µg/mL, which may be rounded to the nearest 1000 µg/mL, i.e., 20000 µg/mL. In certain embodiments, a subject who is 2 years of age or older in the greater than 1.3 m² BSA group may be classified as non-compliant or potentially non-compliant if they exhibit a urinary PAGN level below about 20320 µg/mL or below about 20000 µg/mL. In certain embodiments, the subject may be classified as compliant if they exhibit a urinary PAGN level at or above about 20320 µg/mL or at or above about 20000 µg/mL. In certain embodiments, a dosage of PAA prodrug may be administered to the subject who is 2 years of age or older if the urinary PAGN level for the subject is below the predetermined threshold urinary PAGN level for their BSA group. In certain embodiments, the dosage of PAA prodrug may be an effective dosage of a PAA prodrug as described herein.

One of ordinary skill in the art will recognize that the various embodiments described herein can be combined.

The following examples are provided to better illustrate the claimed invention and are not to be interpreted as limiting the scope of the invention. The invention is defined in the claims.

### Examples

### Example 1: Population PK modeling and dose simulation:

Data from seven different HPN-100 studies were evaluated to develop an improved population PK model for the administration of HPN-100 and NaPBA in the treatment of UCD. The data was derived from four "switchover" studies used to compare HPN-100 and NaPBA (UP1204-003, HPN-100-005, HPN-100-006, HPN-100-012) plus three additional studies (HPN-100-005 safety extension, HPN-100-0007, and HPN-100-012 safety extension). Overall, 114 subjects were included in the dosage analysis. These 114 subjects are estimated to represent approximately 40% of all adult patients and 20-25% of all pediatric patients in the United States receiving NaPBA.

A previously developed population PK model was used as a starting point. That previous model had been developed using data from three of the crossover studies (UP1204-003, HPN-100-005, HPN-100-006). In the previous model, patients age 17 and younger were assigned a unique set of parameters versus adults for estimating conversion of HPN-100 and NaPBA to nitrogen scavenging products. In the present analysis, candidate models used BSA rather than patient age to determine whether this resulted in improved model fit. BSA was evaluated in two different ways: 1) assigning a BSA cutoff of 1.3 m², with patients having a BSA at or below 1.3 m² assigned pediatric presystematic parameters and patients having a BSA above 1.3 m² assigned adult presystematic parameters, and 2) applying BSA/1.73 directly to alpha and beta presystematic parameters to allow continuous scaling of the parameters based on body size to reflect changes in presystematic conversion as a patient grows. The BSA cutoff of 1.3 m² was chosen because it represents the approximate demarcation between children (ages 6 to 11 years) and adolescents (ages 12 to 17 years). Empirically, this cutoff was found to correctly categorize children versus adolescents for all but one patient out of 17.

The resultant updated population PK model was used to perform dosing simulations to evaluate the impact of dose on plasma PAA concentrations. A variety of dosing simulations were utilized, including scenarios designed to simulate patients under age 6 years and to compare mg/kg versus g/m² for patients 20 kg and under. At least 1,500 virtual patients were generated for each dosing scenario. Simulations utilized PBA-equivalent doses of 1) the upper NaPBA labeled dose (13 g/m²/day, maximum 20 g/day) and 2) 50% of the lower NaPBA labeled dose (4.98 g/m²/day) for patients over 20 kg, or 1) 600 mg/kg/day and 2) 225 mg/kg/day for patients 20 kg and under. All daily doses were capped at a PBA equivalent to 20 g NaPBA, the highest labeled dosage for NaPBA. All simulations assumed dosing with meals three times per day.

The four crossover studies used in the present model includes 3,214 measurable data points from 79 patients. This included 1,100 and 1,042 plasma data points (PBA, PAA, and PAGN) for HPN-100 and NaPBA, respectively, from 53 adult UCD patients (UP 1204-003 and HPN-100-006), and 335 and 296 plasma data points for HPN-100 and NaPBA, respectively, from 26 pediatric UCD patients (HPN-100-005 and HPN-100-012). The adult subjects also provided 202 and 197 urinary PAGN measurements for HPN-100 and NaPBA, respectively, while the pediatric patients provided 21 urinary PAGN measurements for both drugs.

Dosing based on BSA (g/m²) was found to produce less variability and slightly lower AUC exposures compared to mg/kg dosing for patients 20 kg or less. Figure 2A shows the actual dosage range (as g PBA per day) for each of the seven studies, while Figure 2B shows the dosage range for each study normalized by BSA (g/m² PBA per day). Figures 3 and 4 show actual and normalized dosage ranges across all studies broken down by age group. Normalized dosage information for the various age groups is summarized in detail in Table 1.

**Table 1:**

| | **<2 years** | **2-5 years** | **6-11 years** | **12-18 years** | **Adult** | **All subjects** |
|---|---|---|---|---|---|---|
| **N** | 7 | 16 | 17 | 9 | 65 | 114 |
| **Mean (g/m²/day PBA)** | 7.33 | 7.53 | 8.89 | 8.09 | 6.92 | 7.42 |
| **Median (g/m²/day PBA)** | 6.65 | 7.76 | 8.98 | 7.32 | 7.13 | 7.43 |
| **Standard deviation (SD)** | 3.18 | 2.5 | 2.7 | 3.45 | 2.78 | 2.85 |
| **% coefficient of variation (CV%)** | 43 | 33 | 30 | 43 | 40 | 38 |
| **25th% (g/m²/day PBA)** | 5.82 | 6.19 | 7.22 | 6.2 | 5.31 | 5.68 |
| **75th% (g/m²/day PBA)** | 8.49 | 8.81 | 10.47 | 11.17 | 8.69 | 9 |
| **Minimum (g/m²/day PBA)** | 2.95 | 1.09 | 2.2 | 2.04 | 0.67 | 0.67 |
| **Maximum (g/m²/day PBA)** | 13.11 | 11.21 | 13.97 | 13.36 | 14.27 | 14.27 |
| **BSA Median** | 0.46 | 0.68 | 0.97 | 1.5 | 1.75 | 1.53 |
| **BSA Range** | 0.32-0.56 | 0.54-0.9 | 0.76-1.27 | 1.26-2.02 | 1.29-2.55 | 0.32-2.55 |

As seen in Table 1 and Figures 3 and 4, the mean dosage varied between the adult and pediatric subjects, and further varied between the various pediatric subgroups. As seen in Figure 3, pediatric subjects age 6 to 17 received a higher normalized dosage of PBA than adult subjects despite receiving a lower actual dosage. Similarly, Figure 4 shows that subjects age 6 to 11 years received a higher normalized dosage than both other pediatric subjects and adult subjects. This data suggest that previous dosing strategies for HPN-100 in which the same dosage per body weight was administered across all age groups may not be appropriate. Instead, subjects under the age of six should receive a lower dosage than older pediatric subjects.

As shown in Figure 4 and Table 2, subjects with a BSA below the cutoff of 1.3 m² received a higher normalized dosage of PBA than subjects at or above the cutoff, despite receiving a lower actual dosage.

**Table 2:**

| | **<1.3 m²** | **≥1.3 m²** |
|---|---|---|
| **N** | 43 | 71 |
| **Mean** | 8.02 | 7.05 |
| **(g/m²/day PBA)** | | |
| **Median** | 8.35 | 7.18 |
| **(g/m²/day PBA)** | | |
| **Standard deviation** | 2.91 | 2.77 |
| **(SD)** | | |
| **% coefficient of variation** | 36 | 39 |
| **(CV%)** | | |
| **25th%** | 6.25 | 5.33 |
| **(g/m²/day PBA)** | | |
| **75th%** | 9.9 | 8.79 |
| **(g/m²/day PBA)** | | |
| **Minimum** | 1.09 | 0.67 |
| **(g/m²/day PBA)** | | |
| **Maximum** | 13.97 | 14.27 |
| **(g/m²/day PBA)** | | |
| **Median age** | 5 | 26 |
| **Age range** | 0.17-20 | 12-75 |

This data suggests that children age 12 to 18 years received a normalized dosage more similar to adults (median of 7.32 g/m²/day vs. 7.13 g/m²/day) than to younger children ages 6 to 11 years children (median of 7.32 g/m²/day vs. 8.98 g/m²/day).

### Example 2: Urinary PAGN to evaluate compliance based on dose:

Pediatric subjects under 6 years of age from the HPN-100-012 safety extension study were divided into three dosing groups: less than 6 mL/m², 6-10 mL/m², and greater than 10 mL/m². Spot urine samples were collected from subjects at 0 hours (i.e., after an overnight fast), and urinary PAGN levels were measured. The results are summarized in Table 3 and Figure 6A.

**Table 3:**

| **Dose categories** | <6 mL/m² | 6-10 mL/m² | >10 mL/m² |
|---|---|---|---|
| **Number of samples** | 19 | 54 | 19 |
| **Mean** | 8353 µg/mL | 12313 µg/mL | 16842 µg/mL |
| **SD** | 9450 µg/mL | 9059 µg/mL | 11127 µg/mL |
| **Median** | 3298 µg/mL | 12144 µg/mL | 17089 µg/mL |
| **10th%** | 643 µg/mL | 1288 µg/mL | 264 µg/mL |
| **25th%** | 1256 µg/mL | 3053 µg/mL | 6990 µg/mL |
| **75th%** | 14290 µg/mL | 20796 µg/mL | 26247 µg/mL |
| **90th%** | 20797 µg/mL | 24429 µg/mL | 28084 µg/mL |

Based on these results, a method was developed for assessing compliance with PAA prodrug therapy. This method compared the urinary PAGN level of a subject to a predetermined threshold urinary PAGN level for the subject's dosage group (i.e., <6 mL/m², 6-10 mL/m² or >10 mL/m²). A subject exhibiting urinary PAGN levels below the predetermined threshold urinary PAGN level for their dosage group was classified as non-compliant or potentially non-compliant, and a subject exhibiting PAGN levels at or above the predetermined threshold urinary PAGN level for their dosage group was classified as compliant.

The 25th percentile reading for each dosage group was incorporated as the predetermined threshold urinary PAGN level. For example, the predetermined threshold urinary PAGN level for a subject in the 6 to 10 mL/m² dosage group is 3053 µg/mL (see

Figure 6A, medium grey and Table 3), which was rounded to the nearest 1000 µg/mL, i.e., 3000 µg/mL. Therefore, subjects in the 6 to 10 mL/m² dosage group with a urinary PAGN level below 3000 µg/mL were classified as non-compliant or potentially non-compliant, and subjects in the 6 to 10 mL/m² dosage group with a urinary PAGN level at or above 3000 µg/mL were classified as compliant. Subjects classified as non-compliant or potentially non-compliant may undergo additional compliance evaluation, and/or may be administered one or more future dosages of PAA prodrug under more tightly controlled conditions, for example under a physician's supervision.

### Example 3: Urinary PAGN to evaluate compliance based on age:

Subjects from all studies who had spot urine samples were divided into three age groups: less than 2 years of age; 2 to less than 6 years of age; and 6 years of age and older. Spot urine samples were collected from subjects after an overnight fast, and urinary PAGN levels were measured. The results are summarized in Table 4 and Figure 6B.

**Table 4:**

| **Age Group** | **< 2 years** | **2- < 6 years** | **≥ 6 years** |
|---|---|---|---|
| | **U-PAGN µg/mL** | | |
| **N** | 54 | 219 | 74 |
| **Mean** | 17330 | 13420 | 10103 |
| **Median** | 17229 | 12114 | 7185 |
| **Minimum** | 348 | 119 | 642 |
| **Maximum** | 44298 | 43372 | 60960 |
| **10^{th} Percentile** | 3265 | 1717 | 1564 |
| **25^{th} Percentile** | 8996 | 5146 | 4032 |
| **75^{th} Percentile** | 25019 | 20603 | 13846 |
| **90^{th} Percentile** | 28028 | 27728 | 21633 |

Based on these results, a method was developed for assessing compliance with PAA prodrug therapy based on age. This method compared the urinary PAGN level of a subject to a predetermined threshold urinary PAGN level for the subject's age group (i.e., < 2 years, 2 to < 6 years, or ≥ 6 years). A subject exhibiting urinary PAGN levels below the predetermined threshold urinary PAGN level for their age group was classified as non-compliant or potentially non-compliant, and a subject exhibiting PAGN levels at or above the predetermined threshold urinary PAGN level for their age group was classified as compliant.

The 25th percentile reading for each age group was incorporated as the predetermined threshold urinary PAGN level. For example, the predetermined threshold urinary PAGN level for a subject who is younger than 2 years of age is 8996 µg/mL (see Figure 6B, dark grey and Table 4), which was rounded to the nearest 1000 µg/mL, i.e., 9000 µg/mL. Therefore, subjects in the younger than 2 years of age group with a urinary PAGN level below 9000 µg/mL were classified as non-compliant or potentially non-compliant, and subjects in the younger than 2 years of age group with a urinary PAGN level at or above 9000 µg/mL were classified as compliant. Subjects classified as non-compliant or potentially non-compliant may undergo additional compliance evaluation, and/or may be administered one or more future dosages of PAA prodrug under more tightly controlled conditions, for example under a physician's supervision.

### Example 4: Urinary PAGN to evaluate compliance based on BSA:

Subjects from all UCD studies and the healthy volunteer study (HPN-100-010) were divided into two groups based on their BSA: less than or equal to 1.3 m² (≤ 1.3 m²) and greater than 1.3 m² (>1.3 m²). Spot urine samples were collected from subjects at 24 hours (i.e., after an overnight fast), and urinary PAGN levels were measured. The results are summarized in Table 5 and shown in Figure 6C and Figures 7A and B.

**Table 5:**

| **BSA Categories** | **BSA ≤ 1.3 m²** | **BSA > 1.3 m²** |
|---|---|---|
| | **U-PAGN µg/mL** | |
| **N** | 87 | 155 |
| **Mean** | 17547.48 | 9058.68 |
| **Minimum** | 119.47 | 1157.49 |
| **5^{th} Percentile** | 1061.84 | 3379.24 |
| **10^{th} Percentile** | 3645.97 | 4078.79 |
| **25^{th} Percentile** | 8390.07 | 5259.01 |
| **50^{th} Percentile** | 17075.13 | 7748.84 |
| **75^{th} Percentile** | 25465.73 | 11916.08 |
| **90^{th} Percentile** | 30830 | 15993.51 |
| **95^{th} Percentile** | 35516.41 | 20320.32 |
| **Maximum** | 44298.26 | 30304.04 |

As shown in Figure 6C and Table 5, subjects with lower BSA who received lower doses of PAA prodrug show higher concentrations of urinary PAGN regardless of the dose they received.

Based on these results, a method was developed for assessing compliance with PAA prodrug therapy based on BSA. This method compared the urinary PAGN level of a subject to a predetermined threshold urinary PAGN level for the subject's BSA group (i.e., BSA ≤ 1.3 m² or BSA > 1.3 m²). A subject exhibiting urinary PAGN levels below the predetermined threshold urinary PAGN level for their BSA group was classified as non-compliant or potentially non-compliant, and a subject exhibiting PAGN levels at or above the predetermined threshold urinary PAGN level for their BSA group was classified as compliant.

The 25th percentile reading for each BSA group was incorporated as the predetermined threshold urinary PAGN level. For example, the predetermined threshold urinary PAGN level for a subject with a BSA of >1.3 m² was 5259 µg/mL (see Figure 6C, dark grey and Table 5), which was rounded to the nearest 1000 µg/mL, i.e., 5000 µg/mL. Therefore, subjects with a BSA of >1.3 m² with a urinary PAGN level below 5000 µg/mL were classified as non-compliant or potentially non-compliant, and subjects with a BSA of >1.3 m² with a urinary PAGN level at or above 5000 µg/mL were classified as compliant. Additionally, based on the 25th percentile reading, the predetermined threshold urinary PAGN level for a subject with a BSA of ≤ 1.3 m² was 8390 µg/mL (see Figure 6C, dark grey and Table 5), which was rounded to the nearest 1000 µg/mL, i.e., 8000 µg/mL. Therefore, subjects with a BSA of ≤ 1.3 m² with a urinary PAGN level below 8000 µg/mL were classified as non-compliant or potentially non-compliant, and subjects with a BSA of ≤ 1.3 m² with a urinary PAGN level at or above 8000 µg/mL were classified as compliant. Subjects classified as non-compliant or potentially non-compliant may undergo additional compliance evaluation, and/or may be administered one or more future dosages of PAA prodrug under more tightly controlled conditions, for example under a physician's supervision.

### Example 5: Urinary PAGN to evaluate compliance based on BSA for subjects 2 years of age and older:

Subjects in Example 4 from all UCD studies and the healthy volunteer study (HPN-100-010) who were 2 years of age and older were divided into two groups based on their BSA: less than or equal to 1.3 m² (≤ 1.3 m²) and greater than 1.3 m² (>1.3 m²). Spot urine samples were collected from subjects at 24 hours (i.e., after an overnight fast), and urinary PAGN levels were measured. The results are summarized in Table 6.

**Table 6:**

| **Age** | **2 years of age and older** | |
|---|---|---|
| **BSA Categories** | **BSA ≤ 1.3 m²** | **BSA > 1.3 m²** |
| | **U-PAGN µg/mL** | |
| **N Obs** | 72 | 156 |
| **N** | 72 | 155 |
| **Mean** | 16186.11 | 9058.68 |
| **Median** | 15869.85 | 7748.84 |
| **Minimum** | 119.4700000 | 1157.49 |
| **5^{th} Percentile** | 621.5940000 | 3379.24 |
| **10^{th} Percentile** | 3479.50 | 4078.79 |
| **25^{th} Percentile** | 7412.48 | 5259.01 |
| **75^{th} Percentile** | 23635.08 | 11916.08 |
| **90^{th} Percentile** | 29835.07 | 15993.51 |
| **95^{th} Percentile** | 33943.83 | 20320.32 |
| **Maximum** | 43372.15 | 30304.04 |

Based on these results, a method was developed for assessing compliance with PAA prodrug therapy based on BSA for subjects who were 2 years of age and older. This method compared the urinary PAGN level of a subject who was 2 years of age and older to a predetermined threshold urinary PAGN level for the subject's BSA group (i.e., BSA ≤ 1.3 m² or BSA > 1.3 m²). Subjects 2 years of age and older exhibiting urinary PAGN levels below the predetermined threshold urinary PAGN level for their BSA group were classified as non-compliant or potentially non-compliant, and subjects 2 years of age and older exhibiting PAGN levels at or above the predetermined threshold urinary PAGN level for their BSA group were classified as compliant.

The 25th percentile reading for each BSA group for subjects 2 years of age and older was incorporated as the predetermined threshold urinary PAGN level. For example, the predetermined threshold urinary PAGN level for a subject who was 2 years of age or older with a BSA of >1.3 m² was 5259 µg/mL, which was rounded to the nearest 1000 µg/mL, i.e., 5000 µg/mL. Therefore, subjects who were 2 years of age or older with a BSA of >1.3 m² with a urinary PAGN level below 5000 µg/mL were classified as non-compliant or potentially non-compliant, and subjects who were 2 years of age or older with a BSA of >1.3 m² with a urinary PAGN level at or above 5000 µg/mL were classified as compliant. Additionally, based on the 25th percentile reading, the predetermined threshold urinary PAGN level for a subject who was 2 years of age or older with a BSA of ≤ 1.3 m² was 7412 µg/mL, which was rounded to the nearest 1000 µg/mL, i.e., 7000 µg/mL. Therefore, subjects who were 2 years of age or older with a BSA of ≤ 1.3 m² with a urinary PAGN level below 7000 µg/mL were classified as non-compliant or potentially non-compliant, and subjects who were 2 years of age or older with a BSA of ≤ 1.3 m² with a urinary PAGN level at or above 7000 µg/mL were classified as compliant. Subjects classified as non-compliant or potentially non-compliant may undergo additional compliance evaluation, and/or may be administered one or more future dosages of PAA prodrug under more tightly controlled conditions, for example under a physician's supervision.

As stated above, the foregoing is merely intended to illustrate various embodiments of the present invention, which is defined in the claims.

### REFERENCES

1. Brusilow Science 207:659 (1980)
2. Brusilow Pediatr Res 29:147 (1991)
3. Diaz Hepatology 57:2171 (2013)
4. Ghabril Clin Pharmacol Drug Dev 2:278 (2013)
5. Lee Mol Genet Metab 100:221 (2010)
6. Lee Presentation at the 2013 American College of Medical Genetics Meeting in Phoenix entitled "Optimizing Ammonia (NH3) Control in Urea Cycle Disorder (UCD) Patients: A Predictive Model"
7. Lichter-Konecki Mol Genet Metab 103:323 (2011)
8. McGuire Hepatology 51:2077 (2010)
9. Mokhtarani Mol Genet Metab 107:308 (2012)
10. Mokhtarani Presentation at the 2013 American College of Medical Genetics Meeting in Phoenix entitled "The Plasma Ratio of Phenylacetic Acid (PAA) to Phenylacetylglutamine (PAGN) a Potential Biomarker for Patients at Risk of Elevated PAA Levels (full article in press in Molecular Genetics and Metabolism"
11. Monteleone J Clin Pharmacol 53:699 (2013)
12. Smith J Pediatr 162:1228 (2013)
13. Rockey Hepatology 56:248A (2012) (full article entitled "Randomized, Double-Blind, Controlled Study of Glycerol Phenylbutyrate in Hepatic Encephalopathy" in press)

## Claims

1. A method of determining an effective dosage of glyceryl tri-[4-phenylbutyrate] for treating a urea cycle disorder in a subject aged 6 to 17 years old in need thereof comprising:
calculating a body surface area (BSA) for the subject; and
comparing the BSA to a predetermined threshold value which is 1.3 m²,
wherein the effective dosage is a first dosage of 7.3 to 8.79 g/m²/day if the BSA is at or above the predetermined threshold value or a second dosage of 9 to 9.9 g/m²/day if the BSA is below the predetermined threshold value.

2. The method of claim 1, wherein the first dosage is 8 g/m²/day.

3. The method of claim 1 or claim 2, wherein the second dosage is 9 to 9.5 g/m²/day.

4. Glyceryl tri-[4-phenylbutyrate] for use in a method for treating a urea cycle disorder (UCD) in a subject aged 6 to 17 years old in need thereof, wherein the method includes the steps of:
calculating a body surface area (BSA) for the subject,
comparing the BSA to a predetermined threshold value which is 1.3 m², and
administering a first dosage of 7.3 to 8.79 g/m²/day of glyceryl tri-[4-phenylbutyrate] if the BSA is at or above the predetermined threshold value or a second dosage of 9 to 9.9 g/m²/day of glyceryl tri-[4-phenylbutyrate] if the BSA is below the predetermined threshold value.

5. Glyceryl tri-[4-phenylbutyrate] for use as in claim 4, wherein the first dosage is 8 g/m²/day.

6. Glyceryl tri-[4-phenylbutyrate] for use as in claim 4 or claim 5, wherein the second dosage is 9 to 9.5 g/m²/day.

7. The method of any of claims 1-3, or glyceryl tri-[4-phenylbutyrate] for use as in any of claims 4-6, wherein the subject has not been treated previously with a PAA prodrug or another nitrogen scavenging drug, wherein the PAA prodrug is selected from the group consisting of glyceryl tri-[4-phenylbutyrate] and phenylbutyrate or a pharmaceutically acceptable salt thereof including sodium phenylbutyrate.

## Patentansprüche

1. Verfahren zur Bestimmung einer wirksamen Dosierung von Glyceryl-Tri-[4-Phenylbutyrat] zur Behandlung einer Harnstoffzyklusstörung bei einem behandlungsbedürftigen Individuum im Alter von 6 bis 17 Jahren umfassend:
Berechnen einer Körperoberfläche (BSA) des Individuums; und
Vergleichen der BSA mit einem vorbestimmten Schwellenwert, der 1,3 m² beträgt,
wobei die wirksame Dosierung eine erste Dosierung von 7,3 bis 8,79 g/m²/Tag, falls die BSA bei oder über dem vorbestimmten Schwellenwert liegt, oder eine zweite Dosierung von 9 bis 9,9 g/m²/Tag, falls die BSA unter dem vorbestimmten Schwellenwert liegt, beträgt.

2. Verfahren nach Anspruch 1, wobei die erste Dosierung 8 g/m²/Tag beträgt.

3. Verfahren nach Anspruch 1 oder Anspruch 2, wobei die zweite Dosierung 9 bis 9,5 g/m²/Tag beträgt.

4. Glyceryl-Tri-[4-Phenylbutyrat] zur Verwendung in einem Verfahren zur Behandlung einer Harnstoffzyklusstörung (UCD) bei einem behandlungsbedürftigen Individuum im Alter von 6 bis 17 Jahren, wobei das Verfahren die folgenden Schritte umfasst:
Berechnen einer Körperoberfläche (BSA) des Individuums,
Vergleichen der BSA mit einem vorbestimmten Schwellenwert, der 1,3 m² beträgt, und
Verabreichen einer ersten Dosierung von 7,3 bis 8,79 g/m²/Tag von Glyceryl-Tri-[4-Phenylbutyrat], falls die BSA bei oder über dem vorbestimmten Schwellenwert liegt, oder einer zweiten Dosierung von 9 bis 9,9 g/m²/Tag von Glyceryl-Tri-[4-Phenylbutyrat], falls die BSA unter dem vorbestimmten Schwellenwert liegt.

5. Glyceryl-Tri-[4-Phenylbutyrat] zur Verwendung nach Anspruch 4, wobei die erste Dosierung 8 g/m²/Tag beträgt.

6. Glyceryl-Tri-[4-Phenylbutyrat] zur Verwendung nach Anspruch 4 oder Anspruch 5, wobei die zweite Dosierung 9 bis 9,5 g/m²/Tag beträgt.

7. Verfahren nach einem der Ansprüche 1 bis 3, oder Glyceryl-Tri-[4-Phenylbutyrat] zur Verwendung nach einem der Ansprüche 4 bis 6, wobei das Individuum nicht zuvor mit einem PAA-Prodrug oder einem anderen Stickstoffbindungsarzneimittel behandelt wurde, wobei das PAA-Prodrug aus der Gruppe ausgewählt ist, die aus Glyceryl-Tri-[4-Phenylbutyrat] und Phenylbutyrat oder einem pharmazeutisch verträglichen Salz davon einschließlich Natriumphenylbutyrat besteht.

## Revendications

1. Procédé de détermination d'une dose efficace du tri-[4-phénylbutyrate] de glycéryle pour traiter un trouble du cycle de l'urée chez un sujet âgé de 6 à 17 ans en ayant besoin, comprenant :
le calcul d'une surface corporelle (BSA) pour le sujet ; et
la comparaison de la BSA à une valeur de seuil prédéterminée qui est 1,3 m²,
dans laquelle la dose efficace est une première dose de 7,3 à 8,79 g/m²/jour si la BSA est égale ou supérieure à la valeur de seuil prédéterminée ou une deuxième dose de 9 à 9,9 g/m²/jour si la BSA est inférieure à la valeur de seuil prédéterminée.

2. Procédé selon la revendication 1, dans lequel la première dose est 8 g/m²/jour.

3. Procédé selon la revendication 1 ou la revendication 2, dans lequel la deuxième dose est 9 à 9,5 g/m²/jour.

4. Tri-[4-phénylbutyrate] de glycéryle pour l'usage dans un procédé de traitement d'un trouble du cycle de l'urée (UCD) chez un sujet âgé de 6 à 17 ans en ayant besoin, le procédé comprenant les étapes :
le calcul d'une surface corporelle (BSA) pour le sujet,
la comparaison de la BSA à une valeur de seuil prédéterminée qui est 1,3 m², et
l'administration d'une première dose de 7,3 à 8,79 g/m²/jour du tri-[4-phénylbutyrate] de glycéryle si la BSA est égale ou supérieure à la valeur de seuil prédéterminée ou d'une deuxième dose de 9 à 9,9 g/m²/jour du tri-[4-phénylbutyrate] de glycéryle si la BSA est inférieure à la valeur de seuil prédéterminée.

5. Tri-[4-phénylbutyrate] de glycéryle pour l'usage selon la revendication 4, dans lequel la première dose est 8 g/m²/jour.

6. Tri-[4-phénylbutyrate] de glycéryle pour l'usage selon la revendication 4 ou la revendication 5, dans lequel la première dose est 9 à 9,5 g/m²/jour.

7. Procédé selon l'une quelconque des revendications 1 à 3, ou tri-[4-phénylbutyrate] de glycéryle pour l'usage selon l'une quelconque des revendications 4 à 6, dans lequel le sujet n'a pas été traité précédemment avec un promédicament de PAA ou un autre médicament épurateur d'azote, dans lequel le promédicament de PAA est choisi dans le groupe comprenant le tri-[4-phénylbutyrate] de glycéryle et le phénylbutyrate ou un de leurs sels pharmaceutiquement acceptables, y compris le phénylbutyrate de sodium.
